# EUROPEAN PATENT APPLICATION

(11) **EP 1 331 276 A1**
(43) Date of publication of application: **30.07.2003**
(21) Application number: 02425030.0
(22) Date of filing: 28.01.2002
(51) Int. Cl.: C12Q 1/68, C12N 1/20

(54) **Method and kit for detecting Gram-positive bacteria**

(71) Applicant: Universita' degli studi di Bologna, 40126 Bologna (IT)
(72) Inventor: Girotti, Stefano, c/o Univ. degli Studi di Bologna, 40126 Bologna (IT)
(74) Representative: Bongiovanni, Simone

(57) **Abstract**

Method and kit for detecting a Gram-positive bacterium, the method including a first enrichment step, which is carried out by homogenizing and incubating a sample in a selective enrichment medium, for example half-Fraser broth or LEB (Listeria enrichment broth), so as to obtain a first intermediate solution, a second enrichment step, which is carried out by inoculating the first intermediate solution into a culture medium, for example BHI (brain heart infusion), so as to obtain a second intermediate solution, a lysis step for destroying the cell walls of any bacterium cells that may be contained in the second intermediate solution, a DNA-amplification step carried out by means of polymerase chain reaction (PCR), for amplification of fragments of DNA of the bacterium, and a detection step for detecting amplified DNA fragments in the presence of an appropriate internal control for the PCR.

## Description

The present invention relates to a method for detecting a Gram-positive bacterium.

The present invention finds advantageous application in the field of microbiological analysis of foodstuffs, to which the ensuing treatment explicitly refers, without any loss of generality.

In the field of microbiological analysis of foodstuffs, it is known that pathogenic bacteria, such as *Listeria monocytogenes*, which is a Gram-positive bacterium, may contaminate a wide range of foodstuffs, in particular dairy products, thus rendering the foodstuffs themselves a fearsome vehicle for the transmission of listeriosis or other illnesses.

For this reason, analytical methods have been proposed which envisage the following steps: inoculation of the samples to be analysed into selective enrichment media (such as Fraser or half-Fraser broth), in order to bring about proliferation of any cells (growth phase) of a bacterium, such as *Listeria monocytogenes*, that may be present; lysis of the walls of the cells of the bacterium thus proliferated by using lysis solutions; and amplification of specific fragments of DNA of the bacterium by means of polymerase chain reaction (PCR) with the aid of a DNA-polymerase enzyme.

The methods of analysis so far proposed present the disadvantage of using lysis solutions containing NaOH, lysozyme/proteinase K or detergents, such as sodium dodecylsulphate (SDS), or other relatively powerful inhibitors of the aforesaid DNA-polymerase enzyme.

For this reason, the above-mentioned analytical methods afford a relatively low reliability, a relatively poor sensitivity, and require relatively long execution times. In this regard, it is important to emphasize that usually the relatively poor sensitivity of the known methods is improved by increasing the time of the growth phase, so as to obtain relatively large colonies of bacterial cells.

An object of the present invention is to provide a method for detecting a Gram-positive bacterium which is free from the drawbacks described above and is, at the same time, of convenient, inexpensive and fast implementation.

Provided in accordance with the present invention is a method for detection of a bacterium as claimed in Claim 1.

According to a preferred embodiment of the method specified above, the bacterium is *Listeria monocytogenes.*

Preferably, in the method specified above, the enrichment step comprises a first enrichment sub-step and a second enrichment sub-step. The first enrichment sub-step is carried out by homogenizing and incubating the sample in a selective enrichment medium so as to obtain an intermediate sample. The second enrichment sub-step is carried out by inoculating the intermediate sample into a culture medium suitable for growth of the bacterium.

The present invention moreover relates to a kit for detection of a bacterium.

Provided according to the present invention is a kit for detecting a bacterium as claimed in Claim 13.

Further characteristics of the present invention will emerge clearly from the ensuing description, which regards possible non-limiting embodiments thereof.

In order to replace the known analytical methods, a kit and a method are provided for detecting a bacterium within a sample.

The kit comprises a selective enrichment medium, such as the half-Fraser broth or the Listeria enrichment broth (LEB), a culture medium suitable for growth of the bacterium, such as brain-heart infusion (BHI) or Tryptone soya and yeast extract broth, a lysis solution, which has a pH of between 3.5 and 9.5, preferably between 7 and 8, and is compatible with DNA-polymerase enzymes in so far as it does not substantially contain NaOH, and lysozyme/proteinase K or SDS or other relatively powerful inhibitors of DNA-polymerase enzymes. Preferably, the lysis solution is an aqueous buffer solution of *tris*-HCl or HEPES (2-[4-(2-hydroxyethyl-1-piperazinyl)]-ethanesulphonic acid) or MOPS (3-(N-morpholine)-propanesulphonic acid sodium salt), at a concentration of between 5 and 50 mM, preferably 20 mM, and KCl, at a concentration of between 20 and 100 mM, preferably 50 mM.

The kit moreover comprises at least one DNA-polymerase enzyme and at least two primers. Preferably, the kit further comprises the triphosphate desoxynucleosides dATP, dGTP, dTTP, and dCTP, at least one DNA fragment for an internal control of the polymerase chain reaction (PCR), at least one loading buffer for electrophoresis, and a fluorescent stain, such as ethidium bromide, that will intercalate into the DNA.

The DNA fragment for internal control of the PCR is, during the PCR, amplified thanks to the DNA-polymerase enzyme and the primers so as to enable verification of whether the PCR has gone through correctly.

The selective enrichment medium, the medium suitable for growth of the bacterium, the lysis solution, the DNA-polymerase, the primers, the triphosphate desoxynucleosides, and the loading buffer, according to various embodiments, may be in liquid form, in particular in an aqueous solution, or in solid form, in particular lyophilised.

According to alternative embodiments, the kit comprises a fluorescent stain, which may be used for a real-time PCR (real-time PCR methods are, for instance, described in EP 0 512 334), or else substrates, which may be used for immunochemical measurements of colour change or fluorescence or chemiluminescence.

The kit moreover comprises a user instruction manual.

During use, the kit is employed following a method that comprises an enrichment step in order to cause proliferation of the cells of the bacterium present in a sample to be analysed, a lysis step in order to break down the walls of the bacterium cells, a DNA-amplification step carried out by means of polymerase chain reaction (PCR), in which at least one fragment of the DNA of the bacterium is amplified, and a detection step for detecting the amplified DNA fragment.

The enrichment step is carried out in such a way that at the end thereof the cells will be in their phase of exponential growth, and thus will be young and have relatively thin cell walls.

In particular, during the enrichment step, the sample to be analysed, or a part thereof, is homegenized and incubated in a selective enrichment medium, such as the half-Fraser broth or the Listeria enrichment broth (LEB), for a time interval of between 16 and 28 hours, preferably between 18 and 24 hours, at a temperature of between 25°C and 40°C, preferably between 30°C and 37°C, to obtain an intermediate sample.

At this point, the intermediate sample, or a part thereof, is inoculated into a culture medium suitable for growth of the bacterium, such as brain-heart infusion (BHI) or Tryptone soya and yeast extract broth, for a time interval of between 4 and 8 hours, preferably between 5 and 6 hours, at a temperature of between 25°C and 40°C, in particular substantially 37°C, to obtain a solution which is then centrifuged. At the end of centrifugation, a sediment is obtained which is next washed with an iso-osmotic solution.

Preferably the weight ratio between the intermediate sample and the culture medium suitable for growth of the bacterium is between 1:10 and 1:1000.

The washed sediment is immersed, during the lysis step, in a lysis solution, which is compatible with DNA-polymerase enzymes and does not inhibit the said DNA-polymerase enzymes, is heated to a temperature of between 80°C and 121°C, preferably 95°C, for a time interval of between 10 and 60 minutes, preferably between 20 and 30 minutes, in particular substantially 25 minutes, and is cooled off very rapidly and then centrifuged. The lysis solution is compatible with the DNA-polymerase enzymes in so far as it does not substantially contain NaOH, lysozyme/proteinase K or SDS, or other relatively powerful inhibitors of the DNA-polymerase enzymes, and preferably has a pH of between 3.5 and 9.5, and in particular between 7 and 8. In particular, the lysis solution is an aqueous buffer solution containing *tris*-HCl, or HEPES or MOPS, at a concentration of between 5 mM and 50 mM, preferably 20 mM, and KCl, at a concentration of between 20 mM and 100 mM, preferably 50 mM.

At the end of the centrifugation step, a supernatant is obtained which is subjected to the DNA-amplification step (by means of polymerase chain reaction - PCR) of a substantially known type, so as to obtain a solution which, during the detection step, is treated using electrophoresis in a substantially known way. Identification of the amplified DNA fragment is, in a known way, performed by staining with ethidium bromide and visualization with ultraviolet (UV) light.

According to different embodiments, the detection step may be performed also by means of real-time-PCR techniques, using a fluorescent stain, or else by means of immunochemical detection, which employs substrates and measures colour change, fluorescence or chemiluminescence.

Further characteristics of the present invention will emerge clearly from the ensuing description of a number of merely illustrative and non-limiting examples. In particular, even though all the examples refer to the detection of *Listeria monocytogenes*, it is believed that the method and kit proposed herein may be used also for other Gram-positive bacteria. In addition, it is important to emphasize that the growth phase may be conducted at temperatures and for time intervals different from the ones indicated in the ensuing examples. The time intervals and temperatures of the growth phase must in any case be chosen in such a way as to favour growth of the bacteria, and in particular in such a way as to obtain, at the end of the growth phase itself, bacteria in a phase of exponential growth.

### Example 1

Four samples of caciocavallo cheese were each added to respective 90 mL of half-Fraser broth (Merck, Darmstadt, Germany) so as to obtain four substantially identical solutions for analysis, each sample having a weight of 10 g.

Four solutions of a culture of *Listeria monocytogenes* were diluted in an aqueous solution containing 0.85 wt% NaCl so as to obtain four contaminated solutions comprising, respectively, 10² cfu/mL, 10¹ cfu/mL, 10⁰ cfu/mL, and 10⁻¹ cfu/mL of *Listeria monocytogenes.*

Of each contaminated solution, 1 mL was homogenized and incubated in a respective solution for analysis at a temperature of 30°C for a time interval of between 22 and 24 hours so as to obtain four intermediate samples.

Of each intermediate sample, 0.1 mL was inoculated into 9.9 mL of BHI (Merck, Darmstadt, Germany) under stirring for 5 hours at a temperature of 37°C to obtain four respective post-enrichment solutions.

Of each post-enrichment solution, 1 mL was centrifuged at 10 000 g for 5 minutes, so as to obtain four sediments. Each sediment was added to a respective aqueous solution (1 mL) containing 0.85 wt% NaCl and was centrifuged at 10 000 g for 5 minutes to obtain four washed sediments. At this point, each washed sediment was added to 0.2 mL of an aqueous solution of *tris-*HCl (20 mM) and KCL (50 mM), the pH of which was substantially 8.4 and which had the function of lysis solution, so as to obtain four suspensions.

Each suspension was heated to a temperature of substantially 95°C for a time interval of substantially 25 minutes. At this point, each suspension was centrifuged at 10 000 g for 5 minutes to obtain four supernatant solutions, each of which was added to a reaction mixture, the pH of which was substantially 8.4, to perform a DNA amplification by means of PCR. The reaction mixture for the PCR was an aqueous solution substantially including *tris*-HCl (20 mM), KCl (50 mM), MgCl₂ (1.5 mM), 200 µM of each triphosphate desoxynucleoside (dATP, dGTP, dTTP, and dCTP; produced by Pharmacia, Upsala, Sweden), 0.6 µM of the inlB-R primer (SEQ ID NO: 1) (Pangallo, D.; Kaclikova, E.; Kuchta, T.; and Drahovska: "Detection of *Listeria monocytogenes* by polymerase chain reaction oriented to in1B gene"; Microbiologica Oct. 2001; 24(4): 333-9), 0.6 µM of the in1B-L primer (SEQ ID NO: 2) (Pangallo, D.; Kaclikova, E.; Kuchta, T.; and Drahovska H.: "Detection of *Listeria monocytogenes* by polymerase chain reaction oriented to in1B gene"; Microbiologica Oct. 2001; 24(4): 333-9), and substantially 25 nkat of Platinum Taq DNA Polymerase (a DNA-polymerase enzyme produced by Life Technologies, Gaithersburg, MD, USA).

The primers inlB-R (SEQ ID NO: 1) and in1B-L (SEQ ID NO: 2) are oriented to the in1B gene (Pangallo, D.; Kaclikova, E.; Kuchta, T.; and Drahovska H.: "Detection of *Listeria monocytogenes* by polymerase chain reaction oriented to in1B gene"; Microbiologica Oct. 2001; 24(4): 333-9).

DNA amplification was performed using an amplification program comprising an initial denaturation at a temperature of 95°C for 2 minutes, in thirty-five cycles, each of which included a denaturation step, at a temperature of 93°C for 45 seconds, a step of cooling to a temperature of 60°C for 45 seconds, and a polymerization step at a temperature of 72°C for 90 seconds. At the end of the thirty-five cycles, a further polymerization step was carried out at a temperature of 72°C for 8 minutes.

Of each solution obtained at the end of the DNA-amplification step, 12 µL were mixed to a loading buffer, were separated by means of gel electrophoresis, the gel containing 1 wt% agarose, were stained with ethidium bromide and visualized by means of UV light. The positive/negative results were read according to the presence/absence of a amplified DNA fragment of 343 bp.

On the basis of the results obtained, a limit of detection of *Listeria monocytogenes* in the caciocavallo cheese of substantially 10° cfu/10 g was calculated.

### Example 2

Four samples of caciocavallo cheese were each added to respective 90 mL of half-Fraser broth so as to obtain four substantially identical solutions for analysis, each sample having a weight of 10 g.

Four solutions of a culture of *Listeria monocytogenes* were diluted in an aqueous solution containing 0.85 wt% NaCl so as to obtain four contaminated solutions comprising, respectively, 10² cfu/mL, 10¹ cfu/mL, 10⁰ cfu/mL, and 10⁻¹ cfu/mL of *Listeria monocytogenes.*

Of each contaminated solution, 1 mL was homogenized and incubated in a respective solution for analysis at a temperature of 30°C for a time interval of between 22 and 24 hours so as to obtain four intermediate samples.

Of each intermediate sample, 0.1 mL was treated in accordance with ISO 11290-1:1996.
On the basis of the results obtained, a limit of detection of *Listeria monocytogenes* in the caciocavallo cheese of 10⁰ cfu/10 g was calculated.

It is important to emphasize that the said detection limit is substantially identical to the detection limit obtained in Example 1.

### Example 3

Four samples of Camembert were treated in the way described in Example 1 for the caciocavallo samples. On the basis of the results obtained, a limit of detection of *Listeria monocytogenes* in the Camembert of 10⁰ cfu/10 g was calculated.

### Example 4

Four samples of Camembert were treated in the way described in Example 2 for the caciocavallo samples. On the basis of the results obtained, a limit of detection of *Listeria monocytogenes* in the Camembert of 10⁰ cfu/10 g was calculated.

It is important to emphasize that the said detection limit is substantially identical to the detection limit obtained in Example 3.

### Example 5

Four samples of Roquefort were treated in the way described in Example 1 for the caciocavallo samples. On the basis of the results obtained, a limit of detection of *Listeria monocytogenes* in the Roquefort of 10⁰ cfu/10 g was calculated.

### Example 6

Four samples of Roquefort were treated in the way described in Example 2 for the caciocavallo samples. On the basis of the results obtained, a limit of detection of *Listeria monocytogenes* in the Roquefort of 10⁰ cfu/10 g was calculated.

It is important to emphasize that the said detection limit is substantially identical to the detection limit obtained in Example 5.

### Example 7

Four samples of mozzarella cheese were treated in the way described in Example 1 for the caciocavallo samples. On the basis of the results obtained, a limit of detection of *Listeria monocytogenes* in the mozzarella of 10⁰ cfu/10 g was calculated.

### Example 8

Four samples of mozzarella cheese were treated in the way described in Example 2 for the caciocavallo samples. On the basis of the results obtained, a limit of detection of *Listeria monocytogenes* in the Mozzarella of 10⁰ cfu/10 g was calculated.

It is important to emphasize that the said detection limit is substantially identical to the detection limit obtained in Example 7.

### Example 9

Four samples of smoked fish were treated in the way described in Example 1 for the caciocavallo samples. On the basis of the results obtained, a limit of detection of *Listeria monocytogenes* in the smoked fish of 10⁰ cfu/10 g was calculated.

### Example 10

Four samples of smoked fish were treated in the way described in Example 2 for the caciocavallo samples. On the basis of the results obtained, a limit of detection of *Listeria monocytogenes* in the smoked fish of 10⁰ cfu/10 g was calculated.

It is important to emphasize that the said detection limit is substantially identical to the detection limit obtained in Example 9.

### Example 11

Four samples of caciocavallo cheese were each added to respective 225 mL of LEB broth (Merck, Darmstadt, Germany) to obtain four substantially identical solutions for analysis, each sample weighing 25 g.

Four solutions of a culture of *Listeria monocytogenes* were diluted in an aqueous solution containing 0.85 wt% NaCl so as to obtain four contaminated solutions comprising, respectively, 10² cfu/mL, 10¹ cfu/mL, 10⁰ cfu/mL, and 10⁻¹ cfu/mL of *Listeria monocytogenes.*

Of each contaminated solution, 1 mL was homogenized and incubated in a respective solution for analysis at a temperature of 37°C for a time interval of between 22 and 24 hours so as to obtain four intermediate samples.

Of each intermediate sample, 0.1 mL was inoculated into respective 9.9 mL of Tryptone soya and yeast extract broth (Oxoid, Basingstoke, England) under stirring for 6 hours at a temperature of 37°C to obtain four post-enrichment solutions.

Of each post-enrichment solution, 1 mL was treated as described in Example 1.

On the basis of the results obtained, a limit of detection of *Listeria monocytogenes* in the caciocavallo cheese of 10⁰ cfu/25 g was calculated.

### Example 12

Four samples of caciocavallo cheese were each added to respective 225 mL of LEB broth to obtain four substantially identical solutions for analysis, each sample weighing 25 g.

Four solutions of a culture of *Listeria monocykogenes* were diluted in an aqueous solution containing 0.85 wt% NaCl so as to obtain four contaminated solutions comprising, respectively, 10² cfu/mL, 10¹ cfu/mL, 10⁰ cfu/mL, and 10⁻¹ cfu/mL of *Listeria monocytogenes.*

Of each contaminated solution, 1 mL was homogenized and incubated in a respective solution for analysis at a temperature of 37°C for a time interval of between 22 and 24 hours so as to obtain four intermediate samples.

Of each intermediate sample, 0.1 mL was treated in accordance with ISO 10560:1993.

On the basis of the results obtained, a limit of detection of *Listeria monocytogenes* in the caciocavallo cheese of 10⁰ cfu/25 g was calculated.
It is important to emphasize that the said detection limit is substantially identical to the detection limit obtained in Example 11.

### Example 13

Four samples of Camembert were treated in the way described in Example 11 for the caciocavallo samples. On the basis of the results obtained, a limit of detection of *Listeria monocytogenes* in the Camembert of 10⁰ cfu/25 g was calculated.

### Example 14

Four samples of Camembert were treated in the way described in Example 12 for the caciocavallo samples. On the basis of the results obtained, a limit of detection of *Listeria monocytogenes* in the Camembert of 10⁰ cfu/25 g was calculated.

It is important to emphasize that the said detection limit is substantially identical to the detection limit obtained in Example 13.

### Example 15

Four samples of Roquefort were treated in the way described in Example 11 for the caciocavallo samples. On the basis of the results obtained, a limit of detection of *Listeria monocytogenes* in the Roquefort of 10⁰ cfu/25 g was calculated.

### Example 16

Four samples of Roquefort were treated in the way described in Example 12 for the caciocavallo samples. On the basis of the results obtained, a limit of detection of *Listeria monocytogenes* in the Roquefort of 10⁰ cfu/25 g was calculated.

It is important to emphasize that the said detection limit is substantially identical to the detection limit obtained in Example 15.

### Example 17

Four samples of mozzarella cheese were treated in the way described in Example 11 for the caciocavallo samples. On the basis of the results obtained, a limit of detection of *Listeria monocytogenes* in the mozzarella cheese of 10⁰ cfu/25 g was calculated.

### Example 18

Four samples of mozzarella cheese were treated in the way described in Example 12 for the caciocavallo samples. On the basis of the results obtained, a limit of detection of *Listeria monocytogenes* in the mozzarella cheese of 10⁰ cfu/25 g was calculated.

It is important to emphasize that the said detection limit is substantially identical to the detection limit obtained in Example 17.

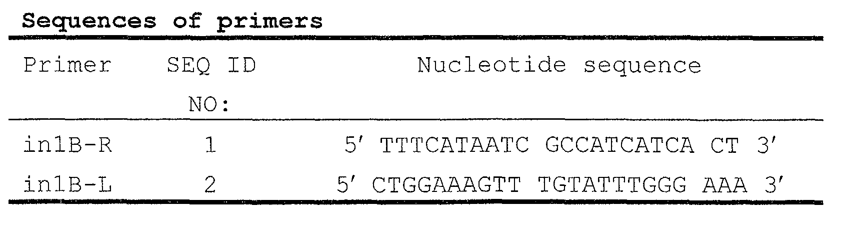

### References cited

ISO 11290-1:1996. Microbiology of food and animal feeding stuffs - Horizontal method for the detection and enumeration of *Listeria monocytogenes*, Part 1: Detection method. International Organization for Standardization, Geneva, 1996.

ISO 10560:1993. Milk and milk products - Detection of *Listeria monocytogenes*. Detection method. International Organization for Standardization, Geneva, 1996.
Pangallo, D.; Kaclikova, E.; Kuchta, T.; and Drahovska H.: "Detection of *Listeria monocytogenes* by polymerase chain reaction oriented to in1B gene"; Microbiologica Oct. 2001; 24(4): 333-9.

## Claims

1. A method for detecting a Gram-positive bacterium within a sample, said method comprising: an enrichment step to cause the cells of the bacterium present in the sample to proliferate; a lysis step to break down the walls of the cells of the bacterium obtained after the enrichment step; a DNA-amplification step (polymerase chain reaction, PCR), in which at least one fragment of the DNA of the bacterium is amplified using at least one DNA-polymerase enzyme; and a detection step to detect the fragment of DNA of the bacterium; the method being **characterized in that** the enrichment step is carried out in such a way that the cells of the bacterium subjected to the lysis step are in a phase of exponential growth; the lysis step being carried out using lysis means which are compatible with the said DNA-polymerase enzyme and to not inhibit the DNA-polymerase enzyme itself.

2. A method according to Claim 1, in which said bacterium is *Listeria monocytogenes.*

3. A method according to Claim 1 or Claim 2, in which said enrichment step comprises a first enrichment sub-step and a second enrichment sub-step; said first enrichment sub-step being carried out by homogenizing and incubating the sample in a selective enrichment medium so as to obtain an intermediate sample; said second enrichment sub-step being carried out by inoculating the intermediate sample into a culture medium suitable for growth of the bacterium.

4. A method according to Claim 3, in which the weight ratio between the intermediate sample and the culture medium suitable for growth of the bacterium is between 1:10 and 1:1000.

5. A method according to Claim 3 or Claim 4, in which said selective enrichment medium is chosen in the group consisting of:
- half-Fraser nutrient broth; and
- Listeria enrichment broth (LEB).

6. A method according to any one of Claims 3 to 5, in which said first enrichment sub-step is carried out for a time interval of between 16 and 28 hours.

7. A method according to any one of Claim 3 to 6, in which said culture medium suitable for growth of the bacterium is chosen in the group consisting of:
- brain heart infusion (BHI); and
- Tryptone soya and yeast extract broth.

8. A method according to any one of Claim 3 to 7, in which said second enrichment sub-step is carried out at a temperature of between 25°C and 40°C for a time interval of between 4 and 8 hours.

9. A method according to any one of the foregoing claims, in which said lysis step is carried out at a pH of substantially between 3.5 -and 9.5, at a temperature of substantially between 80°C and 121°C for a time interval of between 10 and 60 minutes.

10. A method according to any one of the foregoing claims, in which said lysis step is carried out at a pH of between 7 and 8, at a temperature of substantially 95°C for a time interval of substantially 25 minutes.

11. A method according to any one of the foregoing claims, in which said lysis means are chosen in the group consisting of:
- a lysis solution comprising *tris*-HCl and KCl ;
- a lysis solution comprising HEPES and KCl ; and
- a lysis solution comprising MOPS and KCl.

12. A method according to one of the foregoing claims, in which said enrichment step has a duration of between 20 and 36 hours.

13. A kit for detecting a Gram-positive bacterium, said kit comprising: a selective enrichment medium for proliferation of the bacterium; lysis means for lysis of the walls of the cells of the bacterium; at least two primers; and at least one DNA-polymerase enzyme for amplifying at least one fragment of the DNA of the bacterium; the kit being **characterized in that** it comprises at least one culture medium suitable for growth of the bacterium which is used for proliferation of the bacterium; said lysis means being compatible with the said DNA-polymerase enzyme and not inhibiting the DNA-polymerase enzyme itself.

14. A kit according to Claim 12 or Claim 13, in which said bacterium is *Listeria monocytogenes*.

15. A kit according to any one of Claims 12 to 14, comprising the triphosphate desoxynucleosides dATP, dGTP, dTTP, and dCTP.

16. A kit according to any one of Claims 12 to 15, comprising at least one DNA fragment for internal control of the PCR.

17. A kit according to any one of Claims 12 to 16, in which said selective enrichment medium comprises a medium chosen in the group consisting of:
- half-Fraser nutrient broth; and
- Listeria enrichment broth (LEB).

18. A kit according to any one of Claims 12 to 17, in which said medium suitable for growth of the bacterium comprises a medium chosen in the group consisting of:
- brain-heart infusion (BHI); and
- Tryptone soya and yeast extract broth.

19. A kit according to any one of Claims 12 to 18, in which said lysis means are a lysis solution having a pH of substantially between 7 and 8.

20. A kit according to Claim 19, in which said lysis solution is chosen in the group consisting of:
- a buffer solution comprising *tris*-HCl and KCl ;
- a buffer solution comprising HEPES and KCl ; and
- a buffer solution comprising MOPS and KCl.


SEQUENCE LISTING

<110>    Università degli Studi di Bologna

<120>    METHOD AND KIT FOR DETECTING A BACTERIUM

<130>    UNIBO-Girotti1

<140>
<141>

<160>    2

<170>    PatentIn Ver. 2.1

<210>    1
<211>    22
<212>    DNA
<213>    Listeria monocytogenes

<220>
<221>    misc_feature
<222>    (1)..(22)
<223>    primer

<300>
<301>    Pangallo, D.
    Kaclikova, E.
    Kuchta, T.
    Drahovska, H.
<302>    Detection    of    Listeria    monocytogenes    by
polymerase chain reaction oriented to inlB gene
<303>    Microbiologica
<304>    24
<305>    4
<306>    333-339

<307>    2001-10

<400>                                    1 22
tttcataatc gccatcatca ct

<210>    2
<211>    23
<212>    DNA
<213>    Listeria monocytogenes

<220>
<221>    misc_feature
<222>    (1)..(23)
<223>    primer

<300>
<301>    Pangallo, D.
    Kaclikova, E.
    Kuchta, T.
    Drahovska, H.
<302>    Detection of Listeria monocytogenes by
polymerase chain reaction oriented to in1B gene
<303>    Microbiologica
<304>    24
<305>    4
<306>    333-339
<307>    2001-10

<400>                                    2 23
ctggaaagtt tgtatttggg aaa